(19) Europäisches Patentamt
European Patent Office
Office européen des brevets

(11) Publication number: **0 068 498**
**B1**

(12) **EUROPEAN PATENT SPECIFICATION**

(45) Date of publication of patent specification: **19.12.84**

(51) Int. Cl.³: **C 07 C 29/15**, C 07 C 31/04, C 07 C 31/08, C 07 C 31/20

(21) Application number: **82105783.3**

(22) Date of filing: **29.06.82**

(54) **Process for producing alcohols.**

(30) Priority: **30.06.81 US 279095**

(43) Date of publication of application:
**05.01.83 Bulletin 83/01**

(45) Publication of the grant of the patent:
**19.12.84 Bulletin 84/51**

(84) Designated Contracting States:
**BE DE FR GB IT NL**

(56) References cited:
**EP-A-0 013 008**
**GB-A-2 024 811**
**US-A-4 233 466**
**US-A-4 265 828**
**US-A-4 270 015**
**US-A-4 301 253**

**JOURNAL OF THE AMERICAN CHEMICAL SOCIETY, vol. 103, no. 13, July 1981, pages 3959-3961, Washington, USA, J.F. KNIFTON: "Ethylene glycol from synthesis gas via ruthenium melt catalysis"**
**JOURNAL OF THE AMERICAN CHEMICAL SOCIETY, vol. 101, no. 24, November 1979, pages 7419-7421, Easton, USA, J.S. BRADLEY: "Homogeneous carbon monoxide hydrogenation to methanol catalyzed by *soluble ruthenium complexes*"**

(73) Proprietor: **UNION CARBIDE CORPORATION**
**Old Ridgebury Road**
**Danbury Connecticut 06817 (US)**

(72) Inventor: **Dombek, Bernard Duane**
**1631 Woodvale Drive**
**Charleston West Virginia 25314 (US)**

(74) Representative: **Wuesthoff, Franz, Dr.-Ing. et al**
**Patentanwälte Wuesthoff -v. Pechmann-Behrens-Goetz Schweigerstrasse 2**
**D-8000 München 90 (DE)**

(56) References cited:
**JOURNAL OF THE AMERICAN CHEMICAL SOCIETY, vol. 102, no. 22, October 1980, pages 6855-6857, Washington, USA, B.D. DOMBEK: "Hydrogenation of carbon monoxide to methanol and ethylene glycol by *homogeneous ruthenium catalysts*"**
**JOURNAL OF THE CHEMICAL SOCIETY, Dalton Transactions, no. 9, September 1979, pages *1356-1361*, B.F.G. *JOHNSON* et al.: *"The* triruthenium cluster anion (Ru3H(CO)11)-: Preparation structure and fluxionality"**

**EP 0 068 498 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to an improved process for making ethylene glycol, methanol, and ethanol directly from synthesis gas, i.e., mixtures of hydrogen and carbon monoxide. More particularly, this invention achieves the production of ethylene glycol directly from synthesis gas using a particular synergistic combination of ruthenium carbonyl complexes under process conditions. This invention encompasses a process of producing ethylene glycol, methanol, and ethanol directly from the reaction of synthesis gas in the presence of a stable ruthenium catalyst. The process of this invention is distinctive in the stability of the process, avoiding any significant loss of ruthenium values from reaction, and in the catalyst employed.

Discussion of the prior art

Owing to the limited availability of petroleum sources the cost of producing chemicals from petroleum has been steadily increasing. Many have raised the dire prediction of significant oil shortages in the future. Obviously, a different low cost source is needed which can be converted into the valuable chemicals now derived from petroleum sources. Synthesis gas is one such source which can be effectively utilized in certain circumstances to make chemicals.

The most desirable aspect of synthesis gas is that it can be produced from non-petroleum sources. Synthesis gas is derived by the combustion of any carbonaceous material including coal, or any organic material, such as hydrocarbons, carbohydrates and the like. Synthesis gas has for a long time been considered a desirable starting material for the manufacture of a variety of chemicals. A number of chemicals have been made commercially from synthesis gas. Hydrocarbons have been made by the Fischer-Tropsch catalytic reaction. Methanol is commercially manufactured by a heterogeneous catalytic reaction from synthesis gas. Aldehydes and alcohols are made from the reaction of olefins and synthesis gas. If one could expand the production of chemicals in a commercial manner from synthesis gas then one would not be as presently dependent upon petroleum as the basic raw material even though it is an excellent raw material for making synthesis gas. Accordingly, intense interest in such processes has developed.

U.S. Patent No. 3,833,634, describe a process for preparing glycols by reacting an oxide of carbon with hydrogen using a rhodium carbonyl complex catalyst. The examples of the patent compare the reaction of hydrogen and carbon monoxide in the presence of the desired rhodium containing catalyst and other metals. In Example 9 of the patent, the reaction was attempted with triruthenium dodecacarbonyl as the catalyst using tetrahydrofuran as the solvent with a reaction temperature of 230°C, for 2 hours, and "the product contained no polyhydric alcohol". As will be shown below, Pruett and Walker apparently failed to produce polyhydric alcohols because they did not run at the conditions of reaction long enough and/or with enough ruthenium containing catalyst to achieve reaction to produce at least a detectable amount of a polyhydric alcohol such as ethylene glycol. Unquestionably, ruthenium is not as active a catalyst source to produce glycol as is rhodium under the conditions investigated.

U.S. Patent No. 2,535,060, describes a process for preparing monohydric alcohols by introducing carbon monoxide, hydrogen and a hydroxylated solvent into a reaction vessel and heating the mixture in the presence of a ruthenium-containing substance and an alkaline reagent which controls the pH within the range of 7 to 11.5, at a temperature within the range of 150° to 300°C under a pressure within the range of 200 to 1,000 atmospheres.

Solid ruthenium dioxide is used in Examples 1 and 2 of the aforementioned patent. At column 2, lines 30—33 of the patent, the patentee states his belief that ruthenium dioxide is reduced *in situ* during the reaction. Example 1 compares the use of a number of solutes such as phosphoric acid, acidic phosphate buffer, no solutes at all, ammonia and sodium bicarbonate. In this example the solvent was water. In Example 2, a number of alcohols were characterized as solvents.

In U.S.P. 2,535,060 is stated that ruthenium and its compounds are "specific" in their effect upon this reaction and other catalysts "do *not* lead to straight chain primary alcohols under the conditions of this process". There is no indication that Gresham's process, as operated by him, produced ethylene glycol.

This work should be contrasted with the earlier work described in U.S. Patent No. 2,636,046, wherein the production of polyfunctional oxygen-containing organic products including such compounds as ethylene glycol, glycerine, and the like is described*. This is accomplished by the reaction of hydrogen with carbon monoxide in the presence of a solvent to produce glycol. According to this patent, the reaction of carbon monoxide with hydrogen must be at pressures of above 1,000 atmospheres and "particularly above a minimum of about 1,400 atmospheres" in order to obtain the "polyfunctional oxygen-containing organic compounds... in excellent yield" (column 2, lines 9—17). The patent specifically states at column 2, lines 37—43, that:

---

*Note the evaluation of this work by Rathke and Feder, J. Am. Chem. Soc., *100*, pp. 3623—3625 (May 24, 1978).

"[I]n the hydrogenation of oxides of carbon at pressures of 1,000 atmospheres and below, virtually no polyfunctional compounds are produced. At pressures above 1,000 atmospheres and especially at pressures of about 1,500 to 5,000 atmospheres, preferably 2,000 to 5,000 atmospheres, polyfunctional compounds are obtained."

Though the examples of the patent describe only the use of cobalt catalyst, the patentee, at column 3, line 61, indicates that the catalyst may contain "cobalt, ruthenium, etc." According to the patentee, the most outstanding results are obtained by using a catalyst containing cobalt, especially compounds of cobalt which are soluble in at least one of the ingredients of the reaction mixtures.

According to Roy L. Pruett, *Annals, New York Academy of Sciences*, Vol. 295, pages 239—248 (1977), at page 245, metals other than rhodium were tested to determine the production of ethylene glycol from mixtures of carbon monoxide and hydrogen. These metals include cobalt, ruthenium, copper, manganese, iridium and platinum. Of these metals, only cobalt was found to have a slight activity, citing British Patent 665,698 which corresponds generally to the last mentioned U.S. Patent. Pruett stated that such slight activity with cobalt was "qualitatively" in agreement with the results obtained by Ziesecke, 1952, Brennstoff-Chem, *33*:385.

U.S. Patent No. 2,549,470 is directed to a catalytic process for making monohydric straight chain alcohols and does not mention the production of ethylene glycol. The patent emphasizes the production of straight chain primary hydroxyalkanes having from 3 to 50 or more carbon atoms in the molecule. This, the patent states, is accomplished by introducing hydrogen, carbon monoxide, and a hydroxylated solvent into a reaction vessel, and heating the mixture in the presence of a catalyst of the class consisting of ruthenium metal, ruthenium oxide and ruthenium carbonyl, at a pressure within the range of 200 to 1,000 atmospheres and at a temperature within the range of 100° to 250°C. The liquid hydroxyl-containing reaction medium may be water or alcohol, preferably a primary hydroxyalkane having from 1—10 carbon atoms per molecule. According to the patentee, a substantial proportion of the reaction product usually consists of alcohols containing more than 6 carbon atoms per molecule. The patent goes on to state (column 1, line 50, et seq.):

"The reaction products usually contain virtually no hydrocarbons, acids, esters, or branched-chain alcohols. These results were entirely unexpected, in view of the existing knowledge of the catalytic reaction between carbon monoxide and hydrogen in the presence of alcohols and Group VIII metal catalysts."

According to U.S.P. 2,549,470:

"It should be emphasized here that, under the conditions of temperature, pressure and gas ratios just described, no reaction takes place between carbon monoxide and hydrogen in a liquid medium (water or alcohol) if one of the common group VIII metals, such as cobalt or nickel, is used as the catalyst. This is evidenced by the fact that, using, for example, a cobalt catalyst, no significant drop in pressure is observed when carbon monoxide and hydrogen are contacted under the conditions recited. Ruthenium is thus unexpectedly different from these related metals." (Column 4, lines 19—30.)

The numbered examples indicate an apparent preference for making normal-monohydric alcohols, with the proportion of pentane soluble to pentane insoluble alcohol being at least 2:1. In one example, starting at the bottom of column 6 of the above mentioned patent, the solvent employed is characterized as a carboxylic acid or anhydride rather than the neutral hydroxylated solvents which were described in the other examples. This comparative example demonstrated that in a process operated at 200°C for 18 hours using pressures maintained in the range of 300—950 atmospheres by repressurizing periodically with synthesis gas, there was produced a reaction product containing "a large quantity of wax." According to the author, 40.55 parts of esters boiling from 59°C at atmospheric pressure to 150°C at 116 millimeters pressure were obtained and this can be compared to the wax obtained in the amount of 37.06 parts. In that particular example, the patentee appears to have demonstrated that when the hydroxylated solvent is not employed, the amount of wax essentially equals the amount of pentane soluble alcohol products obtained. This is supported by the statement at column 2 of U.S.P. 2,535,060 which refers to U.S.P. 2,549,470.

At column 3, lines 54 of U.S.P. 2,549,470 the influence that pressure has on the course of the reaction, is described. With pressures up to about 150 atmospheres the reaction products are only hydrocarbons. This appears to be in accord with recent work described in German Patent Application (Offenlegungsschrift) 2,644,185*, only hydrocarbons at such pressures using a ruthenium catalyst, were obtained.

U.S. Patent No. 3,579,566 is concerned with a process of reducing organic acid anhydrides with

---

*See Doyle, et al., *J. of Organometallic Chem., 174*, C55—C58 (1979), who conclude that the process characterized in the German Offenlegungsschrift involved a heterogeneous Fischer-Tropsch reaction.

hydrogen in the presence of a Group VIII noble metal catalyst and a biphyllic ligand of phosphorus, arsenic or antimony. The process of Fenton bears a remarkable similarity to oxo processing conditions to produce aldehydes and alcohols (U.S. Patent No. 3,539,634) except that U.S.P. 3,579,566 fails to supply an olefinic compound to the reaction. In said reaction an acid anhydride, such as acetic acid anhydride, is reduced to ethylidene diacetate in the presence of hydrogen and a rhodium halide or a mixture of palladium chloride and ruthenium trichloride catalyst, provided in combination with triphenylphosphine. Ethylene glycol disacetate is also observed. Carbon monoxide, which is added to some of the examples is described in U.S.P. 3,579,566 at column 2, lines 48—51, as follows: "If desired, a suitable *inert* gas, such as carbon monoxide can also be charged to the reaction zone...". (Emphasis added). Of particular significance is the fact that none of said examples produce a methyl ester, as are produced by the process of EU—A1—0 013 008 discussed below. Another point is that ethylidene diacetate can be thermally cracked to produce vinyl acetate, see column 1, lines 42—44, of U.S.P. 3,579,566. It would seem possible that such occurred in Example 1 and it is further possible that acetic acid added to the vinyl acetate to form ethylene glycol diacetate.

The preparation of methanol, ethylene glycol and ethanol and its carboxylates by reacting hydrogen and carbon monoxide is known from the European application 0 013 008 where an in situ-formed ruthenium carbonyl complex catalyst is used at a reaction temperature of between 50 and 400°C and at a reaction pressure of 34.5 to 1,045 bar abs. Ruthenium carbonyl $Ru_3(CO)_{12}$ is named as initial product for the in situ formation of the catalyst and a promoter is required for the catalyst formation. The most diverse inorganic and organic substances can serve as promoter, including aza-, oxa- and azoxa-Lewis bases, organophosphine iminium compounds, ammonia, hydroxyl amine, hydrazine, carboxylic acids, sulfolane, tetrahydrofuran, alkali- and alkaline earth oxides, -hydroxides, -halides, -carboxylates and many more, the selection of promoters to be used occurring in view of the reaction conditions during the alcohol synthesis. Alkali iodides were used at a reaction temperature of 230°C, an $H_2/CO$ ratio of 1:1 and working pressures of 352, 564 or 880 bar. The promoters serve for solubilisation of the ruthenium compound used. The actual composition of the catalyst system effective during alcohol synthesis was not known.

An interesting exception to the previously reported inactivity of ruthenium catalysts to produce glycol is the high pressure (viz 1650—1750 bars) experiment reported by Fonseca, Jenner, Kiennemann, and Deluzarche, et al., High Pressure Science and Technology, 6th AIRAPT Conference (Chapt. "High Pressure Synthesis of Polyalcohols by Catalytic Hydrogenation of Carbon Monoxide"), pages 733—738 (1979), published by Plenum Press, New York (see also a discussion of the same work in Erdöl und Kohle, *32*, 313 (1979)). The authors report the reaction in tetraglyme of a $CO:H_2$ (1:2 ratio) mixture at 1650—1765 bar, and 230°C using triruthenium dodecacarbonyl and 2-pyridinol as a ligand, both in unstated amounts, for a period of 5 hours. The authors report a percent conversion of 12.9 (unstated basis), and percent yield of polyols of 3 (unstated basis), and percent selectivities as follows: ethylene glycol, 22.9; glycerine, 0; methanol, 16.1. However, in a manuscript entitled "Reactions $CO—H_2$ in Liquid Phase in Presence of Ruthenium Catalysts," by Jenner, Kiennemann, Bagherzadah, and Deluzarche, (React. Kim. Catal. Letters, *15*, 103 (1980).) it is stated that with respect to the above experiment "We never could reproduce the run with $Ru_3(CO)_{12}$ when operating in a vessel which has not been in contact with any rhodium catalyst. We suspect that in the former run, the formation of ethylene glycol was due to catalysis with metallic sediments of rhodium encrusted on the wall of the vessel (we showed that ethylene glycol is produced in appreciable yield with rhodium foam)".* In U.S.P. 4,170,605 the patentees report in Examples I and II the reaction in 1-propanol of synthesis gas ($CO:H_2=1:1$) at (25,000 psig) 1725 bar and at 230°C using ruthenium tris(acetylacetonate) and 2-hydroxypyridine, the latter being the same ligand employed by Fonseca, et al, *supra*, for a period of 2 and 3 hours, respectively. In Example 1 of U.S.P. 4,170,605 the production of 4 grams of product** containing (mole percent basis): ethylene glycol, 57; and methanol 25 is reported. In Example II, 7 grams of product** are reported containing 66 and 16 mole percent of ethylene glycol and methanol, respectively.

W. Keim, et al., (Journal of Catalysis, *61*, (1980)) has reported that reactions of $Ru_3(CO)_{12}$ under very high pressures (2,000 bars) produce mainly methanol and methyl formate, but traces of glycol (0.8 to 1.2 percent of the total products) were also seen. In one experiment a small amount of ethanol was detected. No glycerine was observed in these reactions.

In a recent report (J. Am. Chem. Soc., *101*, 7419 (1979).), J. S. Bradley of Exxon Corporation reported the production of methanol and methyl formate at a selectivity greater than 99% without hydrocarbon products detected, by the reaction of synthesis gas ($H_2:CO=3:2$) under pressures on the order of 1,300 bars and at temperatures around 270°C using a Ru catalyst. Bradley observed that no

---

*This report may be relevant to the reports by Williamson et al, (infra) and Keim et al, (infra).

**Included in the 4 and 7 grams of product are trace amounts of water and methylformate as well as 16 mole percent (Example I) and 15 mole percent (Example II) of propylformate. The latter compound would appear to be derived from 1-propanol initially present in the reaction mixture, rather than a synthesis gas-derived product.

ethanol, ethylene glycol, or acetates formed. Compare this result with that found by Pruett and Walker, *supra*, and the work of Fonseca, et al. and U.S.P. 4,170,605., *supra*.

As pointed out above, ethylene glycol can be produced directly from a mixture of hydrogen and carbon monoxide using a rhodium carbonyl complex as a catalyst. The literature describes (U.S. Patent No. 3,957,857, that a desirable rhodium compound can be in the form of a rhodium carbonyl cluster compound, particularly one which exhibits a particular 3-band infrared spectral pattern. There has been a substantial amount of work done on the formation of ethylene glycol from mixtures of hydrogen and carbon monoxide in the presence of rhodium carbonyl clusters, such as is described in United States Patent Nos. 3,833,634; 3,878,214; 3,878,290; 3,878,292; etc. to name but a few.

The above discussion provides a characterisation of the technology heretofore published or filed upon which relates to the direct production of ethylene glycol from mixtures of carbon monoxide and hydrogen or the production of monohydric alcohols from the direct reaction of hydrogen and carbon monoxide in the presence of a ruthenium catalyst. For the purposes of the discussion and descriptions contained herein, mixtures of hydrogen and carbon monoxide, regardless of the amount of each present, will be characterized, for the sake of convenience, as "synthesis gas". Thus, mole ratios of hydrogen to carbon monoxide of e.g. 40 to 1 and 0.05 to 1 are arbitrarily classified as "synthesis gas". Where the molar ratio of one or the other is significant to the invention herein described, then specific reference to the desired molar ratio will be made.

## Brief description of the Figures

Fig. 1 depicts the infrared spectrum of PPN $[Ru(CO)_3I_3]$, (PPN designates bis[triphenylphosphine]-iminium) prior to use in the process.

Fig. 2 depicts the infrared spectrum of PPN $[HRu_3(CO)_{11}]$, hereinafter discussed, prior to use in the process.

Fig. 3 depicts the infrared spectrum of a 2:1 molar mixture of PPN $[HRu_3(CO)_{11}]$ and PPN $[Ru(CO)_3I_3]$, respectively, prior to use in the process according to this invention.

Fig. 4 depicts the infrared spectrum of a catalytic mixture according to this invention obtained from $Ru_3(CO)_{12}$ and sodium iodide after being employed in the process (as employed in Example 1, hereinafter discussed).

Fig. 5 depicts the infrared spectrum of a reaction mixture after the process is carried out wherein a mixture PPN $[HRu_3(CO)_{11}]$ and PPN $[Ru_3(CO)_3I_3]$ was employed in a 2:1 molar ratio (as employed in Example 4, hereinafter discussed).

Fig. 6 and Fig. 7 depict the relationship between the ratio of moles of $Ru(CO)_3I_3^-$ to moles of $HRu_3(CO)_{11}^-$ and the rate of formation of ethylene glycol. (Table II.)

## Summary of the invention

The process of this invention relates to the production of ethylene glycol in a homogeneous liquid phase reaction by initially providing a synergistic mixture of a ruthenium carbonyl iodide-containing complex and a ruthenium carbonyl hydride complex. The ruthenium catalyst employed in the process is indicated by the presence of two ruthenium carbonyl complexes, i.e., $Ru(CO)_3I_3^-$ and $HRu_3(CO)_{11}^-$, which constitute a synergistic combination indicating the ruthenium catalyst which is characterized by an infrared spectrum characterized by three significant infrared bands at $2100\pm10$, $2015\pm10$ and $1990\pm10$ $cm^{-1}$.

## Detailed description of the invention

The process of this invention involves the conversion of synthesis gas, however derived, into a limited variety of valuable alcohol compounds which themselves can be directly consumed or which can be employed as starting materials to make other valuable chemicals. The process of this invention is concerned with making 2 carbon atom alcohols, to wit, ethanol and ethylene glycol and in particular, ethylene glycol. In addition, the process of this invention also produces methanol. The process of this invention is capable of producing predominantly ethylene glycol or predominantly methanol, or predominantly ethanol, or mixtures of them each in large concentrations. The process of this invention provides the capability of a low cost route to methanol, ethanol and ethylene glycol, especially ethylene glycol.

One of the deficiencies of certain of the aforementioned processes for making ethylene glycol from synthesis gas was the utilization of a rhodium carbonyl complex as the catalyst, which processes are dependent on the high price of rhodium. The high cost of rhodium is created by its limited availability and the tremendous demand for it. (For example rhodium presently is employed in catalytic converters which comprises the automotive combustion devices for reducing automotive pollutant emissions.) Thus, a commercial process which uses rhodium as a catalyst is affected by the high capital expense to purchase the metal and the stringent controls needed to limit catalyst losses in order to keep the economics of the process competitive.* Ruthenium, on the other hand, is a precious metal which presently has no significant commercial application. Its present cost is approximately 1/20th,

* See Cornils, et al., Hydrocarbon Processing, June, 1975, pp. 83 to 91.

and less, that of rhodium even though its concentration in the ore from which both are obtained is about the same. Ruthenium has been explored as a catalyst by many, as is shown by the discussed references, *supra*. It has been considered as a hydrogenation catalyst, as a hydroformylation catalyst, as a catalyst to produce a wide range of monohydric alcohols (non-specific as to any of them) exclusive of methanol, as an alcohol homologation catalyst such as for the conversion of methanol to ethanol,** as a high pressure catalyst to selectively produce methanol and methyl formate, and its inactivity as a catalyst to produce glycol has been noted above.

Detailed description of the invention

This process constitutes a relatively low pressure process for selectively converting synthesis gas to such valuable chemicals as ethylene glycol, ethanol and methanol. Also produced by the process of this invention are glycerol (i.e. glycerine), 1,2-propylene glycol, 1-propanol and methyl formate. However, the process of this invention is mainly concerned with the production of ethylene glycol (the most valued product) and to a lesser extent ethanol and methanol, since they are produced in significantly greater amounts than the other products. The process of this invention is accomplished by the presence of a synergistic combination of two ruthenium carbonyl complexes.

The process of this invention is carried out with a synergistic mixture of ruthenium carbonyl complexes present in a solvent, even through such complexes may exist during the reaction in more than one liquid phase. In this sense, the reaction is termed a homogeneous liquid phase reaction. There may be more than one such phase existing in the reaction zone but the ruthenium catalyst, as indicated by the presence of the two ruthenium carbonyl complexes, is always dissolved in at least one of such phases and is always in a dissolved liquid state. The problem with employing heterogeneous ruthenium catalysis in the reaction zone is that such will induce the Fischer-Tropsch reaction resulting in the formation of hydrocarbons and/or a variety of oxygenated hydrocarbons having a variety of molecular weights with low selectivity to any one compound. In fact, the presence of such products suggests that undissolved ruthenium is present and that a non-homogeneous liquid phase reaction occurred.

The process of this invention involves the initially providing of the synergistic combination of ruthenium carbonyl complexes in the synthesis gas at temperatures, pressures and for a period of time sufficient to produce ethylene glycol. Such conditions are set forth herein. In simplistic and in the broadest terms, the invention comprises the use of the synergistic combination of two ruthenium carbonyl complexes under the reaction conditions (i.e., time, temperature and pressure), preferable in the absence of any other platinum group metals (viz., platinum, palladium, rhodium and iridium),* in an appropriate solvent under a prescribed synthesis gas pressure wherein the synergistic mixture of ruthenium carbonyl complexes $Ru(CO)_3I_3^-$ and $HRu(CO)_{11}^-$ is characterized by an infrared spectrum having three significant infrared bands, to wit, at 2100±10, 2015±10 and 1990±10 $cm^{-1}$.

Further, other infrared bands are usually observed at 2070 $cm^{-1}$, 1955 $cm^{-1}$ and 1720 $cm^{-1}$ (see Figures 1—4). It will be appreciated that the exact position of said infrared hands may be dependent on the solvent employed, counter-ions present/and the presence of ligands but in most cases will be within ±10 $cm^{-1}$ of the above stated value. The reaction conditions comprise (i) a period of time at a temperature and pressure which cause the hydrogen and carbon monoxide to react to produce the desired products (ii) a temperature between 50°C and 400°C and (iii) a pressure between 34.5 (500 psia) and 1035 bar abs. (15,000) psia). The catalyst of this invention is indicated by the presence of three significant infrared bands, and the aforementioned ruthenium containing carbonyl complexes which under the prescribed reaction conditions catalyze the aforementioned reaction between carbon monoxide and hydrogen.

The process of this invention is distinctive in the selection of materials which comprise the homogeneous liquid phase mixture, the reaction parameters and the stability of the ruthenium containing catalyst in most cases, indeed, in all cases studied. As with any technology, this process has undergone evolutionary changes and its further examination will undoubtedly bring more changes, most likely in the form of additional or substitutional steps and/or materials.

It is known that this process may be carried out in the presence of a promoter although selection of the promoter is not clearly understood. A promoter, in the context of this invention, is a material provided to the reaction which provides a promotional effect in that it enhances the production (viz., rate, yield, or efficiency) of any of the products, or it improves the selectivity of the reaction toward ethylene glycol rather than methanol or ethanol, or it improves the selectivity of the reaction to ethanol rather than methanol irrespective of the amount of ethylene glycol produced, or it helps to reduce the loss of ruthenium during the reaction. Typical of the promoters that are believed capable of being employed in the instant process are Lewis base promoters (U.S.P. 4233466) to the extent that such promoter enhances the instant process.

---

**See, for example, U.S. Patents 4,133,966 and 3,285,948; and Japanese Patent Application (Kokai) No. 52—73804/77 (June 21, 1977) [Application No. 50—149391/75 (application date, December 15, 1975)] to Mitsubishi Gas Chemical Industry Company.

*See U.S. Patent 3,989,799, wherein ruthenium is a cation in a mixed metal rhodium-containing carbonyl complex.

The solvent is selected such that the solvent is capable of maintaining the ruthenium carbonyl complex catalyst in the homogeneous liquid phase mixture throughout the reaction. The solvent may possibly provide an additional benefit such as influencing the kinds of ion pairing that exist during the course of the reaction.

Varied reaction conditions, solvents, ligands counter-ions, promoters (if employed), may result in different amounts of the desired products of the process, and different rates, efficiencies and/or yields, but it is believed that although each provides a different and distinct catalytic environment that the synergistic mixture of ruthenium carbonyls aforementioned and the characteristic infrared spectrum will be present.

The aforementioned ruthenium carbonyl catalyst of this invention is also characterized by having an average oxidation state of between −0.2 and 0.25. The average oxidation state of the synergistic combination of the ruthenium carbonyl complexes is calculated by taking the oxidation state of a ruthenium atom in $HRu_3(CO)_{11}^-$ as −1/3 and the oxidation state of a ruthenium atom in $Ru(CO)_3I_3^-$ as +2. Accordingly, the average oxidation state of a 2:1 molar ratio of $HRu_3(CO)_{11}^-$ to $Ru(CO)_3I_3^-$ is zero and such average oxidation state is most preferred. Similarly, as above discussed, ruthenium containing compounds which provide the ruthenium carbonyl catalyst of this invention may be employed.

The ruthenium-containing substances which may be employed to form the catalyst, as characterized by the synergistic ruthenium carbonyl mixture, encompass those which are described, for example, in U.S. Patent No. 2,535,060 at column 2, starting at line 38 to line 48, and ruthenium carbonyl compounds. Ruthenium oxides, such as dioxide, sesquioxide, or tetraoxide, may be converted to the ruthenium carbonyl complex employed in the process of this invention. Ruthenium carbonyl compounds (which include ruthenium carbonyl hydrides or ruthenium carbonyl clusters) are already provided with a carbonyl ligand, and under the conditions of the reaction can be sufficiently changed to achieve the desired catalytic effect. Ruthenium salts such as those of organic acids can be employed to produce the catalyst. In addition to those ruthenium compounds described above, one may employ ruthenium compounds of bidentate ligands, allyl complexes, arene complexes, halides, and alkyl complexes. The choice of ruthenium compounds is varied and not critical so long as the aforementioned synerqistic combination is obtained. A number of ruthenium complexes are known to be more stable to the presence of carbon monoxide than other ruthenium compounds and the skilled worker can determine which particular ruthenium compound might take longer to initiate a reaction than other ruthenium compounds. On that basis, one can select for the purposes of convenience the particular ruthenium compound to be utilized in forming the catalyst. However, ruthenium which is associated with an organic molecule or complexed with carbon monoxide is most readily solubilized so as to provide a readily available source of the ruthenium carbonyl catalyst of this process.

The initially provided synergistic mixture of $Ru(CO)_3I_3^-$ and $HRu_3(CO)_{11}^-$ can be formed by the reaction of $Ru_3(CO)_{12}$ with excess $I^-$ as follows:

$$7/3\ Ru_3(CO)_{12}+3I^-+H_2 \rightarrow 2HRu_3(CO)_{11}^-+Ru(CO)_3I_3^-+3CO$$

Selection of the ruthenium-containing starting material is important if *in situ* formation is desired according to EU—A1—0013008, since it has been observed that use of Ru(II) or Ru(III) halide complexes which do not form the synergistic mixture of $Ru(CO)_3I_3^-$ and $HRu_3(CO)_{11}^-$ do not provide the ruthenium catalyst employed in the process of this invention. However, such Ru(II) or Ru(III) complexes may be converted to the ruthenium catalyst used according to this invention by reaction with appropriate base and an iodide containing compound. For example, if the ruthenium compound is $RuI_3$ the following depicts the conversion of such compound:

$$7RuI_3+21OH^-+25CO+23/2H_2 \rightarrow Ru(CO)_3I_3^-+2HRu_3(CO)_{11}^-+18I^-+21H_2O \qquad (1)$$

The complex $Ru(CO)_3I_3^-$ may be converted to an active Ru catalyst as follows:

$$7Ru(CO_3)_3I_3^-+14OH^-+8H_2+4CO \rightarrow Ru(CO)_3I_3^-+2HRu_3(CO)_{11}^-+18I^-+14H_2O \qquad (2)$$

Similarly, the Ru catalyst according to the invention may be prepared by employing $HRu_3(CO)_{11}^-$ as follows:

$$7/3HRu_3(CO)_{11}^-+7/6I_2+2/3I^- \rightarrow Ru(CO)_3I_3^-+2HRu_3(CO)_{11}^-+2/3CO+1/6H_2 \qquad (3)$$

In addition, the presence of the ruthenium complex catalyst used according to this invention is indicated by a reaction medium having an infrared spectrum characterized by the three significant infrared bands at 2100±10, 2015±10 and 1990±10 $cm^{-1}$.

As characterized by equations (1), (2) and (3) the formation of the catlyst used according to this invention is inhibited by the addition of base (reducing agent) and acid (oxidizing agent) beyond that required to give the ruthenium catalyst.

As characterized above, this process is operated as a homogeneous liquid phase mixture. The

process is typically carried out in a solvent for the catalyst. The solvent is a liquid in which the catalyst components are soluble under the prescribed conditions of the reaction. The solvent may be solid at room temperature but should be at least, in part, be a liquid under the conditions of reaction.

A preferred solvent is a liquid at reaction conditions which is polar or complexes ions. Of the polar solvents those which have a relatively high dielectric constant are more preferred. As for the solvents which complex ions, the desirable solvents are those which under the reaction conditions have the capacity of complexing ions such as available cations. As stated previously, the solvent may provide a promoter component. Solvents having a dielectric constant at 25°C or at its melting temperature, whichever is higher, of greater than 2 are preferred.

Illustrative of suitable polar solvents are, e.g., water, ketones, esters including lactones, amides including lactams, sulfones, sulfoxides, halogenated hydrocarbons, and aromatic hydrocarbons. Illustrative of specific solvents encompassed by the above classes of polar solvents are, for example, aromatic hydrocarbons, e.g., benzene, toluene, xylene, naphthalene, alkylnaphthalene; carboxylic acids such as acetic acid, propionic acid, butyric acid, caproic acid, stearic acid, benzoic acid, cyclohexanecarboxylic acid; see the description of acyl compounds in EU—A1 0013008; ketones such as acetone, methyl ethyl ketone, cyclohexanone, cyclopentanone, etc.; esters such as methyl acetate, ethyl acetate, propyl acetate, butyl acetate, methyl propionate, ethyl butyrate, methyl laurate; anhydrides such as phthalic anhydride, acetic anhydride; lactams such as N-alkyl caprolactams, such as N-methylcaprolactam; N-alkyl pyrrolidinones such as N-methyl pyrrolidinone; cyclic ureas such as N,N'-dimethylimidazolidone; polyols such as ethylene glycol, glycerine, erythritol, polyalkylene glycol containing 2 to 10000 repeating units; lactones such as gamma-butyrolactone; halogenated hydrocarbons such as chlorobenzene, chloroform, methylene chloride, 2,2-dichloropropane; amides such as dimethylformamide, dimethylacetamide, hexamethyl- phosphoramide; sulfones such as sulfolane, dimethylsulfone; the substituted sulfolanes sulfoxides such as dimethylsulfoxide, or diphenyl sulfoxide.

Illustrative of suitable complexing solvents are the ethers, and cryptands. Illustrative of specific solvents encompassed by the above classes of complexing solvents are, for example, ethers such as tetrahydrofuran, tetrahydropyran, diethyl ether, 1,2-dimethoxybenzene, 1,2-diethoxybenzene, the mono and dialkyl ethers of alkylene and polyalkylene glycols, such as ethylene glycol, of 1,2-propylene glycol, of 1,2-butylene glycol, of diethylene glycol, of di-1,2-propylene glycol, of triethylene glycol, of pentaethylene glycol (such as triglyme, tetraglyme and pentaglyme), of di-1,2-butylene glycol, of oxyethylene-oxypropylene glycols, preferably those in which the alkylene group contains 2 and/or 3 carbon atoms in the divalent moiety, such as ethylene and 1,2-propylene; the cryptands (U.S. Patent No. 4,111,975) as promoters and the crown ethers (U.S. Patent No. 4,162,261) as solvents in that case.

The choice of solvent in any particular case can be a complex decision. For example, the carboxylic acids, if employed, are also reactive with ethylene glycol, methanol and ethanol products, to produce ethylene glycol dicarboxylates, methyl carboxylates, and ethyl carboxylates. These carboxylates can be readily hydrolysed to produce the alcohol products. This is not necessarily an uneconomical method to produce such products.

An important class of solvents contemplated in the practice of this invention is a mixture of the aforementioned polar solvents and the complexing solvents. Various polar solvents mixed with other polar or complexing solvents are contemplated to provide enhanced results either in terms of rates, selectivity, conversions and/or yields of one or more of the desired products. Which mixtures will achieve what result has not been determined. Combinations of, e.g., sulfolane with crown ethers, lactones, amides or ureas are contemplated as potentially useful. Combinations of, e.g., crown ethers with lactones, amides, and ureas are contemplated as potentially useful.

The iodide containing compounds employed herein may comprise most any iodide containing compound, including such compounds as iodide salts of metals such alkali metals, alkaline earth metals, cobalt diiodide, or iron (II) iodide. Organic iodide containing compounds may also be employed, e.g. bis(triphenylphosphine)iminium iodide; tetramethylammonium iodide, triethylammonium iodide; pyridinium iodide; tetra-n-propylammonium iodide; tetra-n-butylammonium iodide; tetraphenylphosphonium iodide; tetraphenylarsonium iodide; tetra-n-butylphosphonium iodide; and phenyltrimethylammonium iodide. The addition of such iodide salts is beneficial to provide the formation of ethylene glycol at a substantial rate. Generally, an increase in the concentration of iodide promoter increases the overall rate to ethylene glycol although the selectivity to glycol may decrease.

It is believed that the process may be carried out in the presence of a promoter although selection of the promoter is not clearly understood. A promoter, in the context of this invention, is a material provided to the reaction which provides a promotional effect in that it enhances the production (viz., rate, yield, or efficiency) of any of the products, or it improves the selectivity of the reaction toward the products.

The promoter can be any material used in miniscule quantities to a material employed in maximum quantities the effectiveness of which will in large measure be dependent upon the reaction conditions selected. Representative of the promoters employed in the instant process are iodide containing compounds. It is believed that other promoters may also be employed.

Though the process of this invention is capable of providing a combination of ethylene glycol, ethanol and methanol, in many instances one or more of them is formed as a minor component only.

Because ethylene glycol is the most valued of the products, its production obviously makes this process attractive. By the same reasoning, ethanol's higher market value than methanol also enhances the commercial attractiveness of this process. A process which produces the same amount of ethylene glycol and produces more ethanol will have more commercial attractiveness, assuming all other factors are equal.

The relative amounts of carbon monoxide and hydrogen which are initially present in the reaction mixture can be varied over a wide range. In general, the molar ratio of $CO:H_2$ is in the range of from 40:1 to 1:40, suitably from 20:1 to 1:20, and preferably from 10:1 to 1:10. It is to be understood, however, that molar ratios outside the broadest of these ranges may be employed. Substances or reaction mixtures which give rise to the formation of carbon monoxide and hydrogen under the reaction conditions may be employed instead of the mixtures comprising carbon monoxide and hydrogen which are used in preferred embodiments in the practice of the invention. For instance, the product alcohols are contemplated as obtainable by using mixtures containing carbon dioxide and hydrogen. Mixtures of carbon dioxide, carbon monoxide and hydrogen can also be employed. If desired, the reaction mixture can comprise steam and carbon monoxide.

The quantity of catalyst employed is not narrowly critical and can vary over a wide range. In general, the process is desirably conducted in the presence of a catalytically effective quantity of the active ruthenium species which gives a suitable and reasonable reaction rate. The presence of the catalytic species is indicated by the presence of two ruthenium carbonyl complexes, i.e. $Ru(CO)_3I_3^-$ and $HRu_3(CO)_{11}^-$. It has been observed that the rate of ethylene glycol formation is related to the ratio of these complexes such that although their combined presence indicates the presence of the active ruthenium catalyst, the rate to ethylene glycol increases if the mole ratio of $Ru(CO)_3I_3^-$ to $HRu_3(CO)_{11}^-$ is between 0.01 and 2, preferably between 0.2 and 1. The reaction can proceed when employing as little as about $1\times10^{-6}$ weight percent, and even lesser amounts, of ruthenium based on the total weight of reaction mixture (i.e., the liquid phase mixture). The upper concentration limit can be quite high, e.g., about 30 weight percent ruthenium, and higher, and the realistic upper limit in practicing the invention appears to be dictated and controlled more by economics in view of the cost of ruthenium. Since the rate of conversion of synthesis gas may be dependent upon the concentration of ruthenium employed, higher concentrations achieving higher rates, then large concentrations may prove to be a most desirable embodiment of this invention. Depending on various factors such as the promoter (if employed), the partial pressures of carbon monoxide and hydrogen, the total operative pressure of the system, the operative temperature, the choice of solvent, and other considerations, a catalyst concentration of from $1\times10^{-3}$ to 20 weight percent ruthenium (contained in the complex catalyst) based on the total weight of reaction mixture, is generally desirable in the practice of the invention.

The temperature which may be employed in practicing the process may vary over a wide range of elevated temperatures. In general, the process can be conducted at a temperature between 50°C and 400°C and higher. At the lower end of the temperature range the rate of reaction to desired product becomes markedly slow. At the upper temperature range, catalyst, solvent, or promoter instability may occur. Notwithstanding these factors, reaction will continue and the alcohols and/or their derivatives will be produced. Additionally, one should take notice of the equilibrium reaction for forming ethylene glycol:

$$2CO+3H_2{=}HOCH_2CH_2OH$$

At relatively high temperatures the equilibrium increasingly favors the left hand side of the equation. To drive the reaction to the formation of increased quantities of ethylene glycol, higher partial pressures of carbon monoxide and hydrogen are required. Processes based on correspondingly higher operative pressures, however, do not represent preferred embodiments of the invention in view of the high investment costs associated with erecting chemical plants which utilize high pressure utilities and the necessity of fabricating equipment capable of withstanding such enormous pressures. Preferred temperatures are between 100°C and 350°C., and most desirably between 150°C and 300°C.

The process is suitably effected over a wide superatmospheric pressure range. At pressures below 34.5 bar abs. (500 psia) the rate of desired product formation is quite slow, and consequently, relatively faster reaction rates and/or higher conversions to the desired products can be obtained by employing higher pressures, e.g., pressures of at least 69 bar abs. (1,000 psia). Effecting the process below 1035 bar abs. (15,000), especially below 690 bar abs. (10,000 psia), results in significant cost advantages which are associated with lower pressure equipment requirements and operating costs. A suitable pressure range is from 34.5 bar abs. (500 psia) to 828 bar abs. (12,000 psia). The pressure referred to above represent the total pressure of hydrogen and carbon monoxide.

The process is effected for a period of time sufficient to produce the desired alcohol products and/or derivatives thereof. In general, the residence time to produce the desired products can vary from minutes to a number of hours, e.g., from a few minutes to 24 hours, and longer. It is readily appreciated that the residence period (time) will be influenced to a significant extent by the reaction temperature, the concentration and choice of promoter and ruthenium source, the total gas pressure and the partial pressure exerted by its components, the concentration and choice of solvent, and other factors. The

synthesis of the desired product(s) by the reaction of hydrogen with carbon monoxide is suitably conducted under operative condition which give reasonable reaction rates and/or conversions.

The process can be executed in a batch, semi-continuous, or continuous fashion. The reaction can be conducted in a single reaction zone or a plurality of reaction zones, in series or in parallel, or it may be conducted intermittently or continuously in an elongated tubular zone or series of such zones. The material of construction should be such that it is inert during the reaction and the fabrication of the equipment should be able to withstand the reaction temperature and pressure. The reaction zone can be fitted with internal and/or external heat exchanger(s) to thus control undue temperature fluctuations, or to prevent possible "run-away" reaction temperatures due to the exothermic nature of the reaction. In preferred embodiments of the invention, agitation means to vary the degree of mixing of the reaction mixture can be suitably employed. Mixing induced by vibration, shaker, stirrer, rotatory, oscillation or ultrasonic, are all illustrative of the types of agitation means which are contemplated. Such means are available and well-known to the art. The catalyst may be initially introduced into the reaction zone batchwise, or it may be continuously or intermittently introduced into such zone during the course of the synthesis reaction. Means to introduce and/or adjust the reactants, either intermittently or continuously, into the reaction zone during the course of the reaction can be conveniently utilized in the process especially to maintain the desired molar ratios of and the partial pressures exerted by the reactants.

As intimated previously, the operative conditions can be adjusted to optimize the conversion of the desired product and/or the economics of the process. In a continuous process, for instance, when it is preferred to operate at relatively low conversions, it is generally desirable to recirculate unreacted synthesis gas with/without make-up carbon monoxide and hydrogen to the reactor. Recovery of the desired product can be achieved by methods well-known in the art such as by distillation, fractionation, extraction, and the like. A fraction comprising ruthenium complexes, generally contained in byproducts and/or the solvent, can be recycled to the reaction zone, if desired. All or a portion of such fraction can be removed for recovery of the ruthenium values or regeneration thereof, if necessary.

Many embodiments of the ruthenium carbonyl complexes, promoter and solvent combinations encompassed by this invention are sufficiently stable to allow repeated use of the ruthenium carbonyl complexes. This is especially noted when the promoter is an alkali metal halide, particularly and preferably an alkali metal iodide. For example, the process of this invention can be continuously operated in a pressure reactor into which is continuously fed synthesis gas. The velocity of the synthesis gas is sufficient to strip products of the reaction out of the reactor leaving behind in the reactor the synergistic combination of the ruthenium carbonyl complexes, promoter and solvent combination. The products are separated from the unreacted synthesis gas and the synthesis gas is recycled to the reactor. The products, in this embodiment, are recovered free of ruthenium, promoter, if employed, and solvent. In this embodiment, the catalyst need not be removed from the reactor to a recovery zone for separating product. Thus a catalyst treatment step is avoided. The examples below depict batch reactions; however, the above continuous gas recycle process can be operated in a similar manner. That is, the batch reactor simulates the continuous reactor except for the gas sparging and continuous gas recycle.

Experimental procedure

The following procedure was employed in the examples recorded below.

A 150 ml capacity stainless steel reactor capable of withstanding pressures up to 3,000 bar was charged with a mixture of solvent, catalyst precursor, and optionally a promoter, as indicated below. The reactor was sealed and charged with carbon monoxide to a pressure of 34.5 bar. In some cases the gaseous contents of the reactor are vented to remove oxygen. In these cases the reactor is then repressurized to about 34.5 bar. (This venting procedure may be repeated if desired.)

Heat was then applied to the reactor and its contents, (initially at about 55°C or as otherwise indicated); when the temperature of the mixture inside the reactor reached the designated reaction temperature, as measured by a suitably placed thermocouple, addition of carbon monoxide and hydrogen ($H_2$:CO equals the designated mole ratio) was made to bring the pressure to the specified reaction pressure. The temperature was maintained at the desired value for the reported time period. During this period of time, additional carbon monoxide and hydrogen were added whenever the pressure inside the reactor dropped by more than about 34.5 bar over the entire reaction period.

After the reaction period, the reaction vessel was cooled to room temperature, the reaction vessel vented and the reaction products removed. Analysis of the reaction mixture was made by gas chromatographic methods.

The various rates set forth in the following examples are average rates for the particular product and are determined by measuring the net production of product for the reaction period and assuming a nominal reaction volume of 75 ml. In the following examples, the following procedure was employed:

The infrared spectra of the reaction mixtures were analyzed by withdrawing a sample from a sample bottle blanketed with a nitrogen atmosphere. The sample is placed in an infrared cell having $CaF_2$ windows separated by a 0.1 mm spacer. If necessary, the sample was diluted with the solvent employed in carrying out the reaction. The infrared spectra were recorded using a Perkin-Elmer 281B

infrared spectrophotometer with an infrared cell containing reactor solvent being placed in the reference beam.

Figs. 1—3 show, respectively, the infrared spectra of PPN $[Ru(CO)_3I_3]$ in $CH_2Cl_2$; PPN $[HRu_3(CO)_{11}]$ in $CH_2Cl_2$; and of a mixture of PPN $[HRu_3(CO)_{11}]$ and PPN $[Ru(CO)_3I_3]$ at a 2:1 molar ratio, in sulfolane. Fig. 4 and Fig. 5 show, respectively, the infrared spectra of reaction mixtures (after catalysis) from Examples 1 and 4.

Examples 1—12

The following examples were carried out to demonstrate the ruthenium carbonyl catalyst employed in the process of the invention as indicated by the presence of a synergistic mixture of $Ru(CO)_3I_3^-$ and $HRu(CO)_{11}^-$. In each example, as set forth in Table I, the indicated ruthenium carbonyl complex was employed according to the above described experimental procedure. The process conditions, number of millimoles of ruthenium carbonyl employed, rate of formation of ethylene glycol, rate of formation of methanol and milligram atoms of ruthenium are set forth in Table I.

Examples 13—25

The following examples were carried out to determine the ratio of $Ru(CO)_3I_3^-$ to $HRu_3(CO)_{11}^-$ to be employed in the process. Examples 14 to 19, inclusive, were carried out by employing 1.72 millimoles of PPN $[HRu_3(CO)_{11}]$ while varying the amount of PPN $[Ru(CO)_3I_3]$ as shown for examples 14 to 19 in Table II. Examples 20 to 25, inclusive, were carried out by employing 0.86 millimoles of PPN $[Ru(CO)_3I_3]$ while varying the amount of PPN $[HRu_3(CO)_{11}]$ as shown in Table II.

The results of examples 13 to 25 are graphically displayed in Figures 7 and 8.

TABLE I[a]

| Example | Complex | mmoles | Ru, mg-atom | EG Rate | MeOH Rate |
|---|---|---|---|---|---|
| 1 | $Ru_3(CO)_{12}$ | 2.0 | 6.0 | 0.38 | 2.28 |
| 2 | $PPN[HRu_3(CO)_{11}]$ | 2.0 | 6.0 | 0.10 | 1.64 |
| 3 | $PPN[HRu_3(CO)_3I_3]$ | 6.0 | 6.0 | 0 | 0 |
| 4 | $PPN[HRu_3(CO)_{11}]$<br>$PPN[Ru(CO)_3I_3]$ | 1.72<br>0.86 | 6.0 | 0.41 | 2.92 |
| 5 | $PPN[HRu_3(CO)_{11}]$<br>$PPN[Ru(CO)_3I_3]$ | 1.72<br>1.72 | 6.9 | 0.47 | 2.90 |
| 6 | $PPN[HRu_3(CO)_{11}]$<br>$PPN[Ru(CO)_3I_3]$ | 3.44<br>0.86 | 11.2 | 0.48 | 2.92 |
| 7 | $PPN[HRu_3(CO)_{11}]$<br>$PPN[Ru(CO)_3I_3]$ | 1.72<br>0.86 | 6.0 | 0.17[b] | 1.10[b] |
| 8 | $(PPN)_2[Ru_6C(CO)_{16}]$ | 1.0 | 6.0 | 0.11 | 1.19 |
| 9 | $(PPN)_2[Ru_6C(CO)_{16}]$<br>$PPN[Ru(CO)_3I_3]$ | 0.86<br>0.86 | 6.0 | 0 | 0.16 |
| 10 | $(PPN)_2[Ru_6C(CO)_{16}]$<br>$Ru_3(CO)_{12}$ | 1.0<br>2.0 | 12.0 | 0.45 | 2.55 |
| 11 | $Ru_3(CO)_{12}$ | 1.0 | 3.0 | 0.35 | 1.58 |
| 12 | $Ru_3(CO)_{12}$ | 1.0 | 3.0 | 0.35[c] | 1.91[c] |

[a] Conditions: 75 ml sulfolane solvent, 828 bar 1:1 $H_2/CO$, 230°C, 18 mmoles NaI. Rates are moles/h[1]. (PPN=bis[bis[triphenylphosphine]iminium).

[b] No NaI promoter.

[c] PPNI (18 mmoles) instead of NaI.

TABLE II[a]

| Example | PPN[$Ru(CO)_3I_3$] (mmoles) | PPN[$HRu_3(CO)_{11}$] (mmoles) | Total Ru mg-atoms | EG Rate moles/h | MeOH Rate moles/h |
|---|---|---|---|---|---|
| 13 | — | — | 6.00[b] | 0.55 | 4.62 |
| 14 | 0.21 | 1.72 | 5.37 | 0.18 | 2.60 |
| 15 | 0.42 | 1.72 | 5.59 | 0.24 | 2.95 |
| 16 | 0.86 | 1.72 | 6.00 | 0.53 | 4.67 |
| 17 | 1.72 | 1.72 | 6.88 | 0.54 | 5.55 |
| 18 | 3.44 | 1.72 | 8.60 | 0.19 | 2.84 |
| 19 | 6.88 | 1.72 | 12.04 | 0.02 | 0.08 |
| 20 | 0.86 | 0.21 | 1.49 | 0.01 | 0.10 |
| 21 | 0.86 | 0.43 | 2.15 | 0.02 | 0.11 |
| 22 | 0.86 | 0.86 | 3.44 | 0.11 | 1.66 |
| 23 | 0.86 | 1.72 | 6.00 | 0.53 | 4.67 |
| 24 | 0.86 | 3.44 | 11.18 | 0.48 | 4.21 |
| 25 | 0.86 | 6.88 | 21.50 | 0.41 | 5.43 |

[a] Conditions: 75 ml sulfolane solvent, 828 bar 1:1 $H_2/CO$, 230°, 36 mmoles NaI. (PPN=bis[triphenylphosphine]iminium).
[b] Charged as $Ru_3(CO)_{12}$; standard run.

## Claims

1. The process for making the products methanol, ethylene glycol and ethanol directly from the reaction of hydrogen and carbon monoxide which comprises reacting in a liquid phase a mixture of hydrogen and carbon monoxide in the presence of a solvent and a ruthenium carbonyl complex catalyst at a temperature between 50°C and 400°C and a pressure between 34.5 and 1035 bar abs. characterized that initially provided to the liquid phase is a synergistic combination of $Ru(CO)_3I_3^-$ and $HRu(CO)_{11}^-$ having in there infrared spectrum significant bands at $2100\pm10$ $cm^{-1}$, $2015\pm10$ $cm^{-1}$ and $1990\pm10$ $cm^{-1}$.

2. The ruthenium carbonyl complex catalyst of claim 1 wherein the molar ratio of $Ru(CO)_3I_3^-$ to $HRu(CO)_{11}^-$ is between 0.01 and 2 or between 0.2 and 1.

3. The ruthenium carbonyl complex catalyst of claim 1 or 2 wherein the average oxidation state of ruthenium is between −0.2 and 0.25.

4. The process of claim 1—3 wherein the solvent is polar preferably the solvent complexes ions.

5. The process of claim 4 wherein the solvent is a carboxylic acid and the products formed are corresponding derivative carboxylates.

6. The process of claim 4 wherein the solvent is a sulfone, a lactam or an ether, preferably a crown ether, an alkyl ether of an alkylene glycol, a dialkyl ether of a polyalkylene glycol, tetraglyme or a lactone, preferably butyrolactone.

7. The process of claim 1—5 wherein a promoter of the reaction is provided in the liquid phase.

8. The process of claim 7 wherein an iodide promoter compound is provided in the liquid phase.

9. The process of claim 8 wherein the promoter is an alkali metal iodide preferably sodium iodide, lithium iodide, potassium iodide or cesium iodide.

10. The process of claim 7—9 wherein the amount of iodide promoter provided in the liquid phase ranges from 0.1 mole to $10^6$ moles for each gram atom of ruthenium present.

## Patentansprüche

1. Verfahren zur Herstellung von Methanol, Ethylenglykol und Ethanol direkt durch Umsetzung von Wasserstoff und Kohlenmonoxid, indem in flüssiger Phase ein Gemisch von Wasserstoff und

Kohlenmonoxid in Gegenwart eines Lösungsmittels und eines Rutheniumcarbonylkomplex-Katalysators bei einer Temperatur von 50—400°C unter einem Druck von 34,5—1035 bar abs. umgesetzt wird, dadurch gekennzeichnet, daß man anfangs in der flüssigen Phase eine synergistische Kombination von $Ru(CO)_3I_3^-$ und $HRu(CO)_{11}^-$ mit Banden im IR-Spektrum bei $2100 \pm 10$ $cm^{-1}$, $2015 \pm 10^{-1}$ und $1990 \pm 10^{-1}$ vorsieht.

2. Rutheniumcarbonylkomplex-Katalysator nach Anspruch 1, in welchem das Molverhältnis $Ru(CO)_3I_3^-$ zu $HRu(CO)_{11}^-$ zwischen 0,01 und 2 oder zwischen 0,2 und 1 liegt.

3. Rutheniumcarbonylkomplex-Katalysator nach Anspruch 1 oder 2, worin die mittlere Oxidationsstufe des Rutheniums zwischen —0,2 und 0,25 liegt.

4. Verfahren nach Anspruch 1—3, worin das Lösungsmittel polar ist und bevorzugt Ionen komplexiert.

5. Verfahren nach Anspruch 4, worin das Lösungsmittel eine Carbonsäure ist und die nach diesem Verfahren hergestellten Produkte die davon abgeleiteten Carboxylate sind.

6. Verfahren nach Anspruch 4, worin das Lösungsmittel ein Sulfon, Lactam oder Ether ist, vorzugsweise ein Kronenether, ein Alkylether eines Alkylenglykols, ein Dialkyl-ether eines Polyalkylenglykols, Tetraglym oder ein Lacton, vorzugsweise Butyrolacton.

7. Verfahren nach Anspruch 1—5, worin ein Reaktionspromotor in der flüssigen Phase vorgesehen ist.

8. Verfahren nach Anspruch 7, worin der Reaktionspromotor in der flüssigen Phase eines Iodid ist.

9. Verfahren nach Anspruch 8, worin der Promotor ein Alkaliiodid, vorzugsweise ein Iodid von Natrium, Lithium, Kalium oder Cäsium ist.

10. Verfahren nach Anspruch 7—9, worin die Iodidmenge in der flüssigen Phase, bezogen auf g-Atom Ruthenium, 0,1—$10^6$ Mol beträgt.

## Revendications

1. Procédé d'obtention des produits méthanol, éthylèneglycol et éthanol directement par réaction d'hydrogène et oxyde de carbone, qui consiste à faire réagir en phase liquide un mélange d'hydrogène et d'oxyde de carbone en présence d'un solvant et d'un catalyseur aux complexes de ruthéniumcarbonyle à une température comprise entre 50 et 400°C et à une pression absolue comprise entre 34,5 et 1035 bars, caractérisé en ce qu'on pourvoit initialement la phase liquide d'une association synergique de $Ru(CO)_3I_3^-$ et de $HRu(CO)_{11}^-$ présentant dans leur spectre infrarouge des bandes significatives à $2100 \pm 10$ $cm^{-1}$, $2015 \pm 10$ $cm^{-1}$ et $1990 \pm 10$ $cm^{-1}$.

2. Catalyseur aux complexes de ruthéniumcarbonyle suivant la revendication 1, dans lequel le rapport molaire de $Ru(CO)_3I_3^-$ à $HRu(CO)_{11}^-$ est compris entre 0,01 et 2 ou entre 0,2 et 1.

3. Catalyseur aux complexes de ruthéniumcarbonyle suivant la revendication 1 ou 2, dans lequel l'état moyen d'oxydation du ruthénium se situe entre —0,2 et 0,25.

4. Procédé suivant les revendications 1—3, dans lequel le solvant est polaire, le solvant complexant de préférence les ions.

5. Procédé suivant la revendication 4, dans lequel le solvant est un acide carboxylique et les produits formés sont les dérivés carboxylates correspondants.

6. Procédé suivant la revendication 4 dans lequel le solvant est une sulfone, un lactame ou un éther, de préférence un éther en couronne, un éther d'alkyle d'un alkylèneglycol, un éther de dialkyle d'un polyalkylèneglycol, un tétraglyme ou une lactone, de préférence la butyrolactone.

7. Procédé suivant les revendications 1—5, dans lequel un activateur pour la réaction est prévu dans la phase liquide.

8. Procédé suivant la revendication 7, dans lequel un composé activateur qui est un iodure est prévu dans la phase liquide.

9. Procédé suivant la revendication 8, dans lequel l'activateur est un iodure de métal alcalin, de préférence l'iodure de sodium, l'iodure de lithium, l'iodure de potassium ou l'iodure de césium.

10. Procédé suivant les revendications 7—9, dans lequel la quantité d'iodure activateur prévue dans la phase liquide va de 0,1 à $10^6$ moles pour chaque atome-gramme de ruthénium qui est présent.

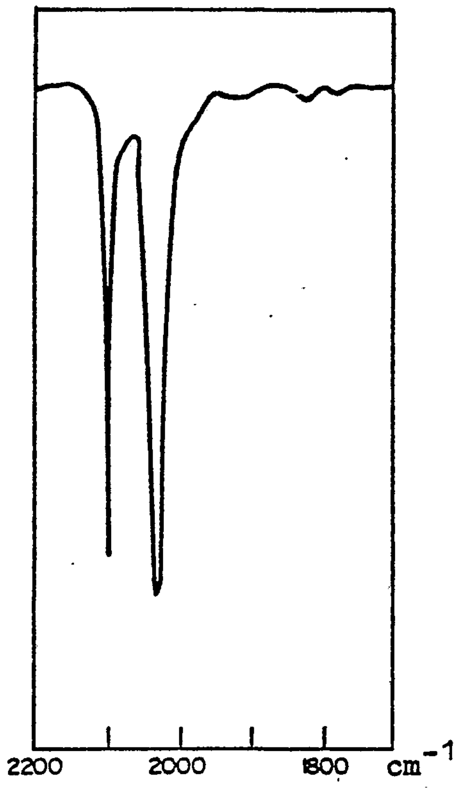


FIG. 1

FIG. 3

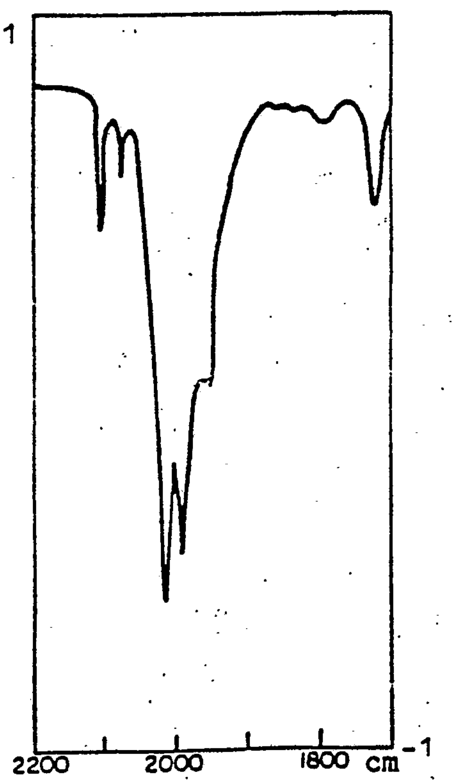

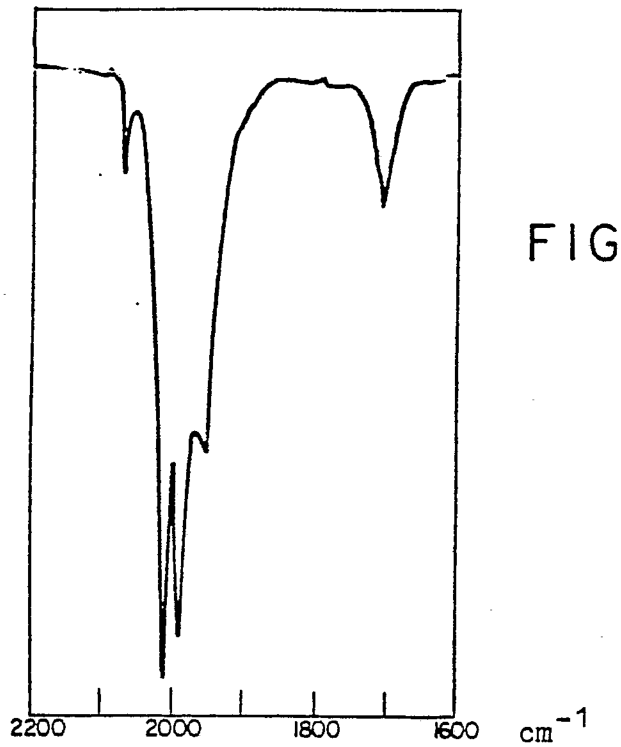


FIG. 2

FIG. 4

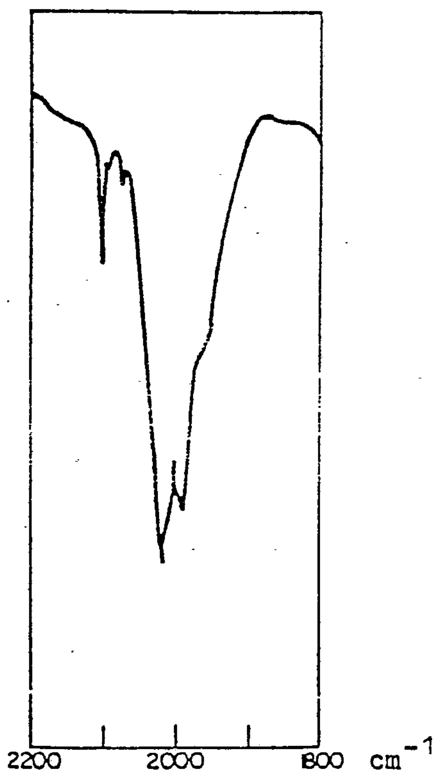

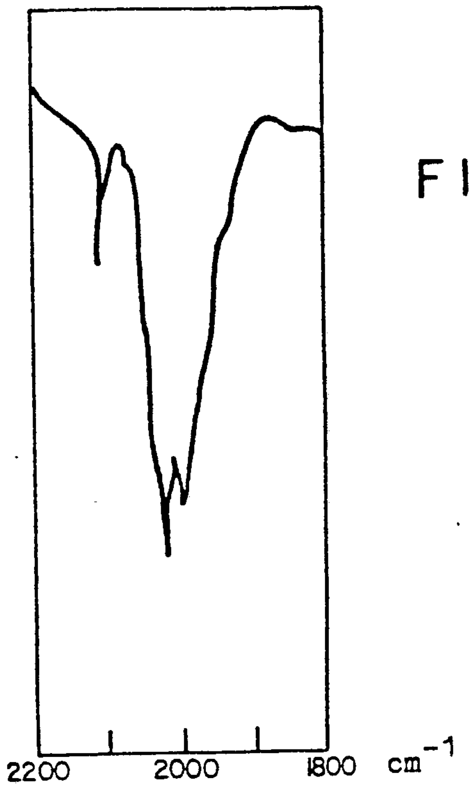


FIG. 5

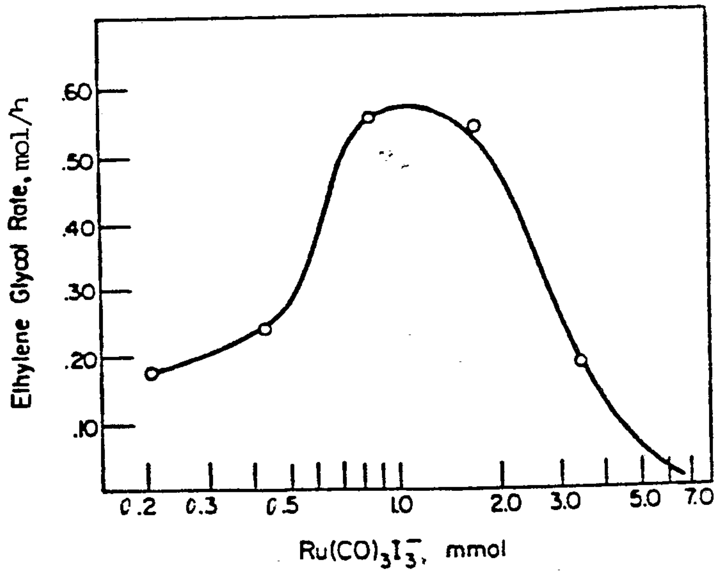
Ethylene Glycol Rate, mol/h
.60
.50
.40
.30
.20
.10
0.2 0.3 0.5 1.0 2.0 3.0 5.0 7.0
$Ru(CO)_3I_3^-$, mmol

FIG. 6

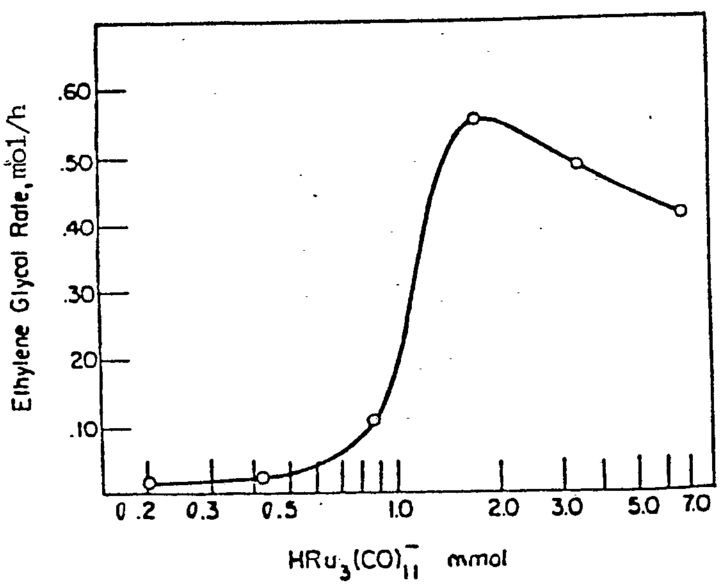
Ethylene Glycol Rate, mol/h
.60
.50
.40
.30
20
.10
0.2 0.3 0.5 1.0 2.0 3.0 5.0 7.0
$HRu_3(CO)_{11}^-$ mmol

FIG. 7